# EUROPEAN PATENT APPLICATION

(11) **EP 4 364 843 A1**
(43) Date of publication of application: **08.05.2024**
(21) Application number: 22833337.3
(22) Date of filing: 01.07.2022
(51) Int. Cl.: B01J 25/00, B01J 37/02, C07C 209/48

(54) **SPONGE COBALT CATALYST COMPOSITION AND METHOD FOR PRODUCING SAME**

(30) Priority: 02.07.2021 JP 2021110950
(71) Applicant: JGC Catalysts and Chemicals Ltd., Kawasaki-shi, Kanagawa 212-0013 (JP)
(72) Inventor: YAMAZAKI, Hirosato, Niigata-shi, Niigata 956-0855 (JP)
(74) Representative: Gille Hrabal Partnerschaftsgesellschaft mbB Patentanwälte
(86) International application number: PCT/JP2022/026528
(87) International publication number: WO 2023/277192

(57) **Abstract**

Overview

Problem to be solved: To provide a sponge cobalt catalyst that exhibits high catalyst activity even in a long-term use.

Means for solving the problem: A sponge cobalt catalyst composition contains water, an oxoacid, and a sponge cobalt catalyst. The oxoacid contains W or Mo, and part or all of the oxoacid is adsorbed to the sponge cobalt catalyst. Selected drawing: None

## Description

### TECHNICAL FIELD

The present invention relates to a sponge cobalt catalyst composition and a method for producing the same.

### BACKGROUND ART

Sponge metal catalysts, which are also referred to as Raney (registered trademark) metal catalysts, are a generic term for catalysts of which main component is a spongy active metal. Further details are described in "Raney Catalyst" written by Teruo Kubomatsu and Shinichiro Komatsu and published by Kyoritsu Shuppan Co., Ltd. in 1971. The sponge metal catalyst is produced by preparing an alloy of a catalytic metal (e.g. nickel, cobalt, copper, iron, silver, and palladium) and to-be-leached metal (e.g. aluminum, silicon, zinc, and magnesium) and leaching (hereinafter also referred to as "activation (activated)" in some cases) the to-be-leached metal from the alloy. The sponge metal catalyst, which has a large number of minute pores created through the above-described production process, is used for various catalytic reactions. A sponge cobalt catalyst, which is included in the sponge metal catalysts, is widely used as a catalyst for hydrogenation reaction (e.g. nitrile hydrogenation).

Patent Literatures 1 and 2 each disclose using the sponge cobalt catalyst in a reaction for hydrogenating adiponitrile to synthesize hexamethylene diamine. Patent Literatures 3 and 4 each disclose using the sponge cobalt catalyst in a reaction for hydrogenating phthalonitrile to synthesize xylylene diamine. Patent Literature 5 discloses using the sponge cobalt catalyst in a reaction for hydrogenating aminoacetonitrile to synthesize ethylene diamine.

### CITATION LIST

### PATENT LITERATURE(S)

Patent Literature 1: JP 2001-302595 A
Patent Literature 2: JP 2002-529227 A
Patent Literature 3: JP S54-41804 A
Patent Literature 4: JP 2013-177346 A
Patent Literature 5: JP 2010-520175 A

### SUMMARY OF THE INVENTION

### PROBLEM(S) TO BE SOLVED BY THE INVENTION

A long-time use of conventional sponge cobalt catalysts is likely to have a decrease in catalyst activity.

An object of the invention is to provide a sponge cobalt catalyst composition containing a sponge cobalt catalyst that exhibits high catalyst activity even in a long-time use and a method for producing the sponge cobalt catalyst composition.

### MEANS FOR SOLVING THE PROBLEM(S)

According to some aspects of the invention, there are provided a sponge cobalt catalyst composition and a method for producing the same, as below.
[1] A sponge cobalt catalyst composition containing: water; an oxoacid; and a sponge cobalt catalyst, in which the oxoacid contains W or Mo, and part or all of the oxoacid is adsorbed to the sponge cobalt catalyst.
[2] The sponge cobalt catalyst composition according to [1], in which a content of the oxoacid containing W and adsorbed to the sponge cobalt catalyst is in a range from 5 mg to 1,200 mg, in terms of W, with respect to 1 kg of the sponge cobalt catalyst.
[3] The sponge cobalt catalyst composition according to [1] or [2], in which a content of the oxoacid containing Mo and adsorbed to the sponge cobalt catalyst is in a range from 5 mg to 1,000 mg, in terms of Mo, with respect to 1 kg of the sponge cobalt catalyst.
[4] The sponge cobalt catalyst composition according to any of [1] to [3], in which the oxoacid is at least one selected from WO₄²⁻, MoO₄²⁻, Mo₇O₂₄⁶⁻, and Mo₈O₂₆⁴⁻.
[5] The sponge cobalt catalyst composition according to any of [1] to [4], in which a molar ratio (W/Co) of W contained in the oxoacid adsorbed to the sponge cobalt catalyst to Co contained in the sponge cobalt catalyst is in a range from 0.00001 to 0.0005.
[6] The sponge cobalt catalyst composition according to any of [1] to [5], in which a molar ratio (Mo/Co) of Mo contained in the oxoacid adsorbed to the sponge cobalt catalyst to Co contained in the sponge cobalt catalyst is in a range from 0.00001 to 0.01.
[7] The sponge cobalt catalyst composition according to any of [1] to [6], in which a molar ratio of Mo to W (Mo/W) contained in the oxoacid adsorbed to the sponge cobalt catalyst is in a range from 1 to 10.
[8] The sponge cobalt catalyst composition according to any of [1] to [7], in which a content of cobalt contained in the sponge cobalt catalyst is in a range from 30 mass% to 70 mass%.
[9] The sponge cobalt catalyst composition according to any of [1] to [8], in which a content of aluminum contained in the sponge cobalt catalyst is in a range from 30 mass% to 70 mass%.
[10] The sponge cobalt catalyst composition according to any of [1] to [9], in which the sponge cobalt catalyst composition is used in a reaction for hydrogenating nitrile.
[11] A method for producing a sponge cobalt catalyst composition, the method including: preparing an alloy containing cobalt and aluminum; removing the aluminum from the alloy to obtain a sponge cobalt catalyst; immersing the sponge cobalt catalyst in water; and adding an oxoacid salt containing W or Mo to the water to adsorb the oxoacid to the sponge cobalt catalyst.

### BRIEF DESCRIPTION OF DRAWINGS

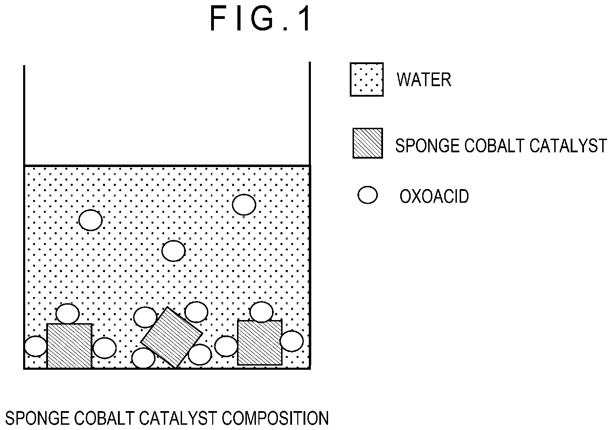

Fig. 1 is an illustration of a sponge cobalt catalyst composition of the invention.

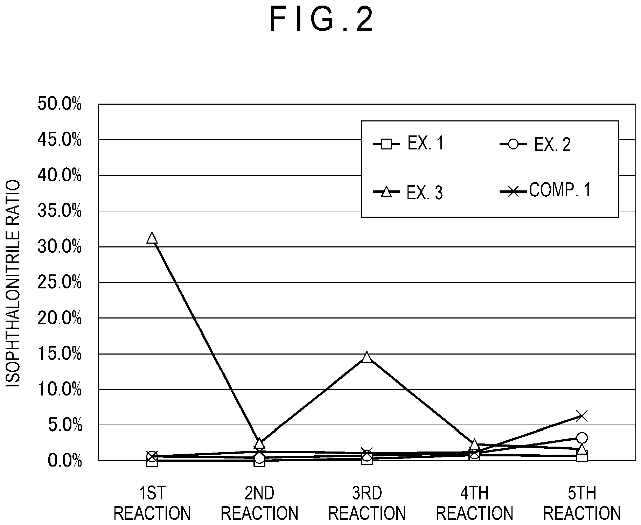

Fig. 2 is a graph showing a relationship between a ratio of isophthalonitrile contained in a reaction solution and the number of reactions in an activity test using sponge cobalt catalysts of Examples 1 to 3 (W adsorbed to the sponge cobalt catalysts: 88 to 534 mg/kg-cat) and Comparative 1 (without W).

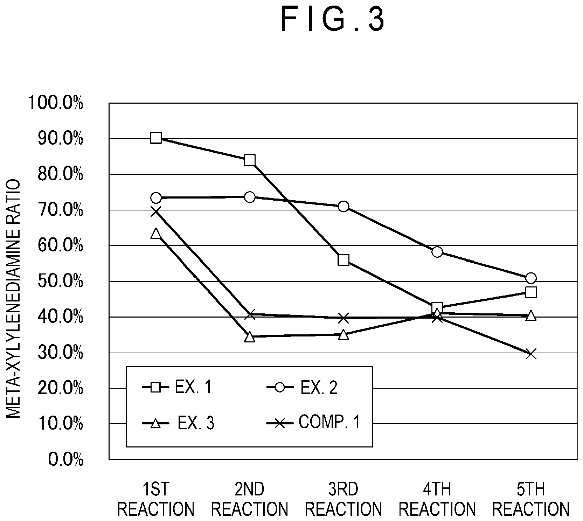

Fig. 3 is a graph of a relationship between a ratio of meta-xylylenediamine contained in a reaction solution and the number of reactions in an activity test using the sponge cobalt catalysts of Examples 1 to 3 (W adsorbed to the sponge cobalt catalysts: 88 to 534 mg/kg-cat) and Comparative 1 (without W).

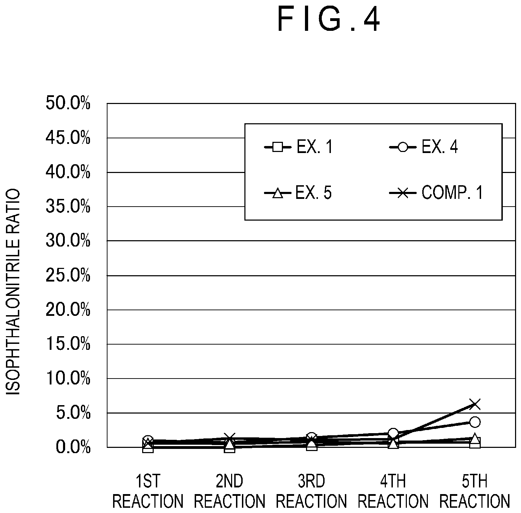

Fig. 4 is a graph showing a relationship between a ratio of isophthalonitrile contained in a reaction solution and the number of reactions in an activity test using the sponge cobalt catalysts of Example 1 (with an oxoacid containing W being adsorbed), Example 4 (with an oxoacid containing Mo being adsorbed), Example 5 (with an oxoacid containing W and an oxoacid containing Mo being adsorbed), and Comparative 1 (without any oxoacid being adsorbed).

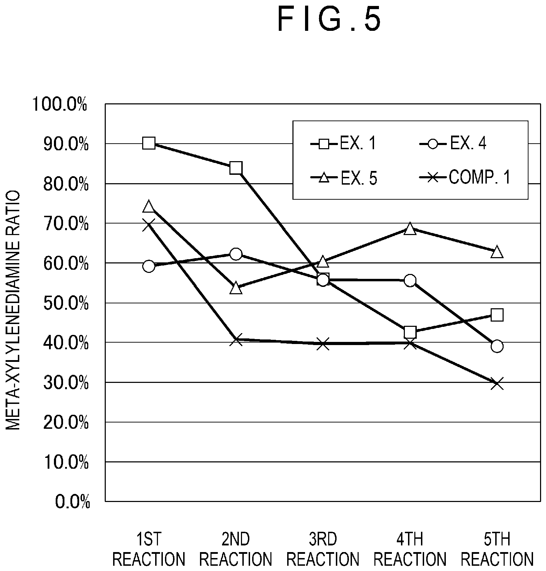

Fig. 5 is a graph showing a relationship between a ratio of meta-xylylenediamine contained in a reaction solution and the number of reactions in an activity test using the sponge cobalt catalysts of Example 1 (with an oxoacid containing W being adsorbed), Example 4 (with an oxoacid containing Mo being adsorbed), Example 5 (with an oxoacid containing W and an oxoacid containing Mo being adsorbed), and Comparative 1 (without any oxoacid being adsorbed).

### DESCRIPTION OF EMBODIMENT(S)

In order to solve the above-described problem(s), an inventor of the invention has studied conditions of surfaces of sponge cobalt catalysts in which catalytic reactions occur. Specifically, the inventor has found that high catalyst activity can be maintained even in a long-term use by adsorbing an oxoacid containing W or Mo to a surface of the sponge cobalt catalyst.

The invention relates to a sponge cobalt catalyst composition containing a sponge cobalt catalyst to which an oxoacid containing W or Mo is adsorbed. The sponge cobalt catalyst composition of the invention (hereinafter also referred to as a "catalyst composition of the invention") will be detailed below.

### Catalyst Composition of the Invention

The catalyst composition of the invention contains water, an oxoacid, and a sponge cobalt catalyst. The surface of the sponge cobalt catalyst is degraded upon being exposed to the air atmosphere. The sponge cobalt catalyst contained in the catalyst composition of the invention is thus present in water. All or part of the oxoacid present in the water is adsorbed to the surface of the sponge cobalt catalyst (see, Fig. 1). It is believed that the sponge cobalt catalyst contained in the catalyst composition of the invention, of which surface is modified by the oxoacid, exhibits excellent catalyst activity even in a long-term use.

The oxoacid contains W (tungsten) or Mo (molybdenum). The oxoacid containing W is preferably WO₄²⁻. Further, the oxoacid containing Mo is preferably MoO₄²⁻, Mo₇O₂₄⁶⁻, Mo₈O₂₆⁴⁻. The sponge cobalt catalyst with the above oxoacid(s) being adsorbed to the surface thereof exhibits excellent catalyst activity even in a long-term use. Further, the sponge cobalt catalyst with an oxoacid containing W and an oxoacid containing Mo being adsorbed thereto exhibits further excellent catalyst activity in a long-term use.

A suitable content of the oxoacid adsorbed to the surface of the sponge cobalt catalyst (also referred to as "oxoacid (adsorbed)") differs between a case where the oxoacid (adsorbed) contains W and a case where the oxoacid (adsorbed) contains Mo. The content of the oxoacid (adsorbed) containing W is preferably in a range from 5 mg to 1,200 mg (in terms of W) with respect to 1 kg of the sponge cobalt catalyst, more preferably in a range from 10 mg to 300 mg, and still more preferably in a range from 20 mg to 200 mg. The content of the oxoacid (adsorbed) containing Mo is preferably in a range from 5 mg to 2,000 mg (in terms of Mo) with respect to 1 kg of the sponge cobalt catalyst, more preferably in a range from 50 mg to 1,500 mg, and still more preferably in a range from 100 mg to 1,200 mg. With the content of the oxoacid (adsorbed) being within the above-described range, the catalyst activity of the sponge cobalt catalyst is likely to be high in a long-term use. The content is calculated based on a value obtained by subtracting an amount of W and/or Mo contained in water from a total amount of W and/or Mo contained in the sponge cobalt catalyst composition of the invention.

A molar ratio (W/Co) of W contained in the oxoacid (adsorbed) to Co contained in the sponge cobalt catalyst is preferably in a range from 0.00001 to 0.0005, more preferably in a range from 0.00002 to 0.0003, and still more preferably in a range from 0.00003 to 0.0001. A molar ratio (Mo/Co) of Mo contained in the oxoacid (adsorbed) to Co contained in the sponge cobalt catalyst is preferably in a range from 0.00001 to 0.01, more preferably in a range from 0.00005 to 0.005, and still more preferably in a range from 0.0001 to 0.003. With the molar ratio being within the above-described range, the sponge cobalt catalyst exhibits excellent catalyst activity even in a long-term use.

When the oxoacid containing W and the oxoacid containing Mo are adsorbed to the surface of the sponge cobalt catalyst, the molar ratio (Mo/W) is preferably in a range from 1 to 10, more preferably in a range from 1 to 7, and still more preferably in a range from 1 to 5. With the molar ratio being within the above-described range, the sponge cobalt catalyst exhibits excellent catalyst activity even in a long-term use.

The sponge cobalt catalyst is spongy by removing part of Al from an alloy containing Co(cobalt) and Al (aluminum). Since the surface area of metal (Co) is large in the spongy structure, the catalyst activity is enhanced.

The Co content in the sponge cobalt catalyst is preferably in a range from 30 mass% to 70 mass%, more preferably in a range from 40 mass% to 60 mass%, and still more preferably in a range from 50 mass% to 60 mass%. With the Co content being within the above-described range, the initial catalyst activity of the sponge cobalt catalyst is likely to be enhanced.

The sponge cobalt catalyst preferably contains Al. The Al content in the catalyst of the invention is preferably in a range from 30 mass% to 70 mass%, more preferably in a range from 40 mass% to 60 mass%, and still more preferably in a range from 50 mass% to 60 mass%.

The sponge cobalt catalyst is preferably in a form of a grain(s). In the invention, a mass of which minor and major axes are less than 1 mm is defined as powder and any other mass than powder is defined as a grain(s). The advantages of the invention are achievable irrespective of whether the sponge cobalt catalyst is in a form of grains or powder, and are effectively achieved when the sponge cobalt catalyst is in a form of grains. An outer surface area of the sponge cobalt catalyst in a form of grains is likely to be smaller than that of the sponge cobalt catalyst in a form of powder, so that the catalyst activity of the grain catalyst easily decreases in a long-time use. The sponge cobalt catalyst with the oxoacid adsorbed to the surface thereof, however, exhibits excellent catalyst activity even in a long-term use. Further, the sponge cobalt catalyst in a form of grains, which is usable as a catalyst for a fixed bed, is easily separable from a product, thereby achieving excellent productivity. The size of the grain (grain size) of the sponge cobalt catalyst is more preferably in a range from 1 mm to 5 mm. The size of the grain can be determined depending on a sieve opening size. For instance, when the catalyst of the invention is sifted using a sieve of which opening is 1 mm, the mass on the sieve is determined to be grains with a size of 1 mm or more and the mass passed through the sieve openings can be determined to be powder with a size of less than 1 mm.

Water serves as a protector for the surface of the sponge cobalt catalyst and also as a medium for adsorbing the oxoacid to the sponge cobalt catalyst. It is thus only necessary for water to be contained to an extent that water covers the surface of the sponge cobalt catalyst. For instance, it is preferable that the sponge cobalt catalyst is fully immersed in water as illustrated in Fig. 1. Accordingly, the amount of water is appropriately controlled depending on the amount of the sponge cobalt catalyst.

The ratio of the oxoacid contained in the water to a total amount of the oxoacid contained in the catalyst composition of the invention is preferably 50% or less, more preferably 40% or less. The oxoacid contained in the catalyst composition of the invention is not totally adsorbed to the sponge cobalt catalyst but may partially remain in the water due to adsorption equilibrium. Water and the sponge cobalt catalyst are separated when the catalyst composition of the invention is in use. The oxoacid contained in water thus causes no serious problems. The ratio of the oxoacid, however, is preferably small to prevent a problem in wastewater treatment or the like.

The pH of the water is preferably 8 or more, more preferably 8.5 or more, and still more preferably 9 or more. With the ph being in the above range, the surface of the sponge cobalt catalyst is positively charged, facilitating the adsorption of negatively charged oxoacid.

The catalyst composition of the invention may contain an ammonium ion or an alkali ion in addition to W, Mo, Co, and Al. These ions are occasionally contained as a counter cation of the oxoacid. Alternatively, these ions are occasionally contained as an ion derived from alkali used in preparing the sponge cobalt catalyst.

The catalyst composition of the invention may contain any other component than W, Mo, Co, Al, the ammonium ion, and the alkali ion in an amount of 10 mass% or less. For instance, an element(s) that is likely to be mixed (e.g. C (carbon), Si (silicon), Mg (magnesium), Ca (calcium)) and an element(s) that serves as a promotor (e.g. nickel, molybdenum, zirconium, copper, chromium, iron, and manganese) are optionally contained. Specifically, the content of the element(s) likely to be mixed is preferably 2 mass% or less, more preferably 1 mass% or less, and still more preferably 0.1 mass% or less. The content of component serving as a promotor is preferably in a range from 0.1 mass% to 10 mass%, more preferably in a range from 0.1 mass% to 5 mass%, and still more preferably in a range from 0.1 mass% to 3 mass%.

The catalyst composition of the invention is usable in a wide range of fields using the cobalt catalyst. For instance, the catalyst composition of the invention is usable as a hydrogenation catalyst. As the hydrogenation reaction, reactions for a carbon-carbon double bond, carbon-carbon triple bond, benzene nucleus, pyridine, carbonyl group, nitro group, nitrile, fatty acid, ester, and the like are known. The catalyst of the invention is suitably usable as a catalyst for nitrile hydrogenation. When the catalyst composition of the invention is used for the above applications, water is removed to take out the sponge cobalt catalyst. At this time, it is believed that the oxoacid adsorbed to the surface of the sponge cobalt catalyst is combined with the counter cation contained in water to form a salt. For instance, when the oxoacid containing W is adsorbed to the sponge cobalt catalyst and a sodium ion is present as the counter cation, it is believed that Na₂WO₄ is formed on the surface of the sponge cobalt catalyst.

### Method for Producing Catalyst Composition of the Invention

A method for producing the catalyst composition of the invention (hereinafter also referred to as a "producing method of the invention") includes an alloy preparation step for preparing an alloy containing Co and Al, an activation step for removing Al from the alloy to obtain the sponge cobalt catalyst, an immersion step for immersing the sponge cobalt catalyst in water, and an adsorption step for adding an oxoacid salt containing W or Mo to the water to adsorb the oxoacid to the sponge cobalt catalyst. The producing method of the invention will be detailed below.

### Alloy Preparation Step

The alloy can be prepared by a typically known method. For instance, the alloy can be prepared by mixing and melting the metal Co and the metal Al. The Co content in the alloy is preferably in a range from 20 mass% to 70 mass%, more preferably in a range from 30 mass% to 60 mass%. The Al content in the alloy is preferably in a range from 30 mass% to 80 mass%, more preferably in a range from 40 mass% to 70 mass%. In the producing method of the invention, part of Al contained in the alloy is removed in the later-described activation step, so that pores are formed at the points occupied by Al, thereby forming a spongy cobalt alloy. In that alloy, the number of pores is likely to increase as the Al content in the alloy is higher, but the strength of the alloy is likely to decrease. The Co content and Al content in the alloy are preferably within the above-described ranges.

The alloy preparation step preferably includes a grain-size adjustment step for adjusting the grain size of the alloy. Specifically, it is preferable that the alloy is pulverized to adjust the size of the grain in a range from 1 mm to 5 mm. Adjusting the grain size of the alloy as described above facilitates the removal of Al in the later-described activation step, thus improving production efficiency.

### Activation Step

A known method is usable to remove Al from the alloy. For instance, the alloy is optionally treated with an alkali solution. When an alkali solution is used, the type of alkali is not specifically limited but typically known alkali such as alkali hydroxide or alkali carbonate is usable. More specifically, sodium hydroxide or potassium hydroxide is preferably used. Further, an amount of alkali in the alkali solution is preferably in a range from 0.01 to 3 times (molar ratio) as much as the Al content in the alloy. With the alkali amount in the alkali solution being within the above-described range, Al in the alloy can be efficiently removed. When the removal of Al is insufficient, Al may be removed to achieve a desired removal level by, for instance, increasing the number of times of the treatment. Further, when Al is to be left in the alloy, the amount of alkali in the alkali solution is preferably in a range from 0.1 times to 1 time as much as the Al amount in the alloy.

In the activation step, a leaching temperature is preferably 10 degrees C or more and less than 100 degrees C, more preferably in a range from 20 degrees C to 90 degrees C. A higher leaching temperature facilitates the leaching of Al. However, when Al is rapidly leached, the activated sponge cobalt catalyst easily collapses. The leaching temperature is thus suitably adjusted depending on the removal rate of Al. Similarly, a leaching time is also suitably adjusted depending on the removal rate of Al. It depends on the amount to be treated, but Al can be properly removed with a leaching time in a range from 0.5 to 12 hours.

### Immersion Step

The sponge cobalt catalyst obtained in the activation step is immersed in water to protect the surface of the sponge cobalt catalyst. Water also serves as a medium for adsorbing the oxoacid to the surface of the sponge cobalt catalyst.

The immersion step may include a washing step for washing the sponge cobalt catalyst. For instance, the sponge cobalt catalyst may be immersed in water after being washed with flowing water. The temperature of water when washing the sponge cobalt catalyst is preferably in a range from 20 degrees C to 60 degrees C, more preferably in a range from 30 degrees C to 50 degrees C. Washing the sponge cobalt catalyst with water having the above temperature range facilitates the removal of soluble impurities contained in the sponge cobalt catalyst.

The immersion step preferably includes a pH adjustment step. Adjusting the pH to 8 or more, preferably 8.5 or more, and still more preferably 9 or more facilitates the adsorption of the oxoacid to the surface of the sponge cobalt catalyst in the later-described adsorption step. A known method is usable to adjust pH. For instance, pH can be adjusted by adding a basic compound such as ammonia, sodium hydroxide, or potassium hydroxide.

### Adsorption Step

A known method is usable as a method for adding, to the water, an oxoacid salt containing W or Mo to adsorb the oxoacid to the sponge cobalt catalyst. For instance, an oxoacid salt such as Na₂WO₄, K₂WO₄, Na₂MoO₄, or Mo₇O₂₄(NH₄)₆ may be added to the water. At this time, the temperature of the aqueous solution containing the oxoacid is preferably in a range from 10 degrees C to 100 degrees C, more preferably in a range from 10 degrees C to 50 degrees C. Further, the time for performing the treatment depends on the amount to be treated, but the oxoacid can be properly adsorbed to the surface of the sponge cobalt catalyst within 1 to 24 hours. Examples

The invention will be described below in further details with reference to Examples. It should however be noted that the scope of the invention is not limited to these Examples.

### Measurement Method or Evaluation Method

Various measurements or evaluations were performed as follows.

### 1. Composition Analysis

A measurement sample was collected in a beaker, heated after adding hydrochloric acid and nitric acid, was dissolved by adding water. Further, after diluting the mixture with water, the contents of Co, Al, W, and Mo were measured using an ICP device (produced by Agilent Technologies Japan, Ltd., 730ICP-OES, inductively coupled plasma emission spectrometry). The amount of the oxoacid containing W or Mo adsorbed to the sponge cobalt catalyst was calculated by subtracting the content of the oxoacid in water from the total content of the oxoacid in the catalyst composition.

### 2. Activity Test

An activity test for hydrogenating nitrile was performed with reference to a method described in Examples of JP S54-41804 A. Specifically, isophthalonitrile (8 g), methanol (24 mL), toluene (96 mL), the measurement sample (sponge cobalt catalyst) (3 g), and sodium hydroxide aqueous solution (50 mass%) (1 g) were charged in a 330 mL autoclave, and reacted at a hydrogen pressure of 8 MPa, a reaction temperature of 70 degrees C, and an agitation rate of 900 rpm for six hours. After the reaction, the measurement sample was removed and the reaction solution was analyzed using gas chromatography. Peaks of isophthalonitrile and meta-xylylenediamine were separated from a resultant chart and ratios of the respective components to all the peak areas included in the chart were determined.

### Example 1

CoAl alloy grains (size: in a range from 1 mm to 5 mm), of which composition was 40 mass% of cobalt and 60 mass% of aluminum, were activated and washed with sodium hydroxide to obtain a sponge cobalt catalyst. The activated sponge cobalt catalyst was immersed in water, and the ph measurement was performed at 25 degrees C. The ph was 10. Subsequently, an aqueous solution containing 150 ppm of Na₂WO₄ with respect to the weight (weight in water) of the sponge cobalt catalyst (Na₂WO₄ · 2H₂O: produced by Wako Pure Chemical Industries, Ltd., special grade reagent) was added at room temperature (herein, the aqueous solution containing 150 ppm of Na₂WO₄ refers to an aqueous solution containing 150 ppm of W derived from Na₂WO₄, which corresponds to 240 mg in equivalent of the amount of Na₂WO₄ added to 1 kg of the sponge cobalt catalyst). Then, after being left for 12 hours or more, the sponge cobalt catalyst composition was obtained. Table 1 shows the charge composition of the resultant sponge cobalt composition and properties thereof obtained through composition analysis and the like. Further, an activity test was performed on the sponge cobalt catalyst separated from the catalyst composition. Table 1 also shows the results. In addition, Figs. 2, 3, 4, and 5 show the results of the activity test.

### Example 2

A sponge cobalt catalyst composition was produced as in Example 1 except that the amount of Na₂WO₄ added to 1 kg of the sponge cobalt catalyst was changed to 150 mg. Table 1 shows the charge composition of the resultant sponge cobalt composition and properties thereof obtained through composition analysis and the like. Further, an activity test was performed on the sponge cobalt catalyst separated from the catalyst composition. Table 1 also shows the results. In addition, Figs. 2 and 3 show the results of the activity test.

### Example 3

A sponge cobalt catalyst composition was produced as in Example 1 except that the amount of Na₂WO₄ added to 1 kg of the sponge cobalt catalyst was changed to 1,600 mg. Table 1 shows the charge composition of the resultant sponge cobalt composition and properties thereof obtained through composition analysis and the like. Further, an activity test was performed on the sponge cobalt catalyst separated from the catalyst composition. Table 1 also shows the results. In addition, Figs. 2 and 3 show the results of the activity test.

### Example 4

A sponge cobalt catalyst composition was produced as in Example 1 except that Mo₇O₂₄(NH₄)₆ (produced by Wako Pure Chemical Industries, Ltd., special grade reagent) was used in place of Na₂WO₄ and the amount of Mo₇O₂₄(NH₄)₆ added to 1 kg of the sponge cobalt catalyst was changed to 240 mg. Table 1 shows the charge composition of the resultant sponge cobalt composition and properties thereof obtained through composition analysis and the like. Further, an activity test was performed on the sponge cobalt catalyst separated from the catalyst composition. Table 1 also shows the results. In addition, Figs. 4 and 5 show the results of the activity test.

### Example 5

A sponge cobalt catalyst composition was produced as in Example 1 except that Na₂WO₄ and Mo₇O₂₄(NH₄)₆ were added and the amount of each of Na₂WO₄ and Mo₇O₂₄(NH₄)₆ added to 1 kg of the sponge cobalt catalyst was changed to 240 mg. Table 1 shows the charge composition of the resultant sponge cobalt composition and properties thereof obtained through composition analysis and the like. Further, an activity test was performed on the sponge cobalt catalyst separated from the catalyst composition. Table 1 also shows the results. In addition, Figs. 4 and 5 show the results of the activity test.

### Example 6

A sponge cobalt catalyst composition was produced as in Example 1 except that K₂WO₄ (produced by Wako Pure Chemical Industries, Ltd., reagent) was used in place of Na₂WO₄. Table 1 shows the charge composition of the resultant sponge cobalt composition and properties thereof obtained through composition analysis and the like. Further, an activity test was performed on the sponge cobalt catalyst separated from the catalyst composition. Table 1 also shows the results.

### Example 7

A sponge cobalt catalyst composition was produced as in Example 4 except that the amount of Mo₇O₂₄(NH₄)₆ added to 1 kg of the sponge cobalt catalyst was changed to 1,000 mg. Table 1 shows the charge composition of the resultant sponge cobalt composition and properties thereof obtained through composition analysis and the like. Further, an activity test was performed on the sponge cobalt catalyst separated from the catalyst composition. Table 1 also shows the results.

### Comparative 1

A sponge cobalt catalyst composition was produced as in Example 1 except that Na₂WO₄ was not added. Table 1 shows the charge composition of the resultant sponge cobalt composition and properties thereof obtained through composition analysis and the like. Further, an activity test was performed on the sponge cobalt catalyst separated from the catalyst composition. Table 1 also shows the results. In addition, Figs. 2, 3, 4, and 5 show the results of the activity test.

**Table 1**

| | | | | Ex. 1 | Ex. 2 | Ex. 3 | Ex. 4 | Ex. 5 | Ex. 6 | Ex. 7 | Comp. 1 |
|---|---|---|---|---|---|---|---|---|---|---|---|
| Charge Composition | Water | Volume | [mL] | 44 | 44 | 44 | 44 | 44 | 44 | 44 | 44 |
| | Sponge Cobalt | Co | [mass%] | 55 | 55 | 55 | 55 | 55 | 55 | 55 | 55 |
| | Catalyst | Al | [mass%] | 45 | 45 | 45 | 45 | 45 | 45 | 45 | 45 |
| | Oxoacid Salt | Na₂WO₄ | [mg/kg-cat] | 240 | 150 | 1600 | - | 240 | - | - | 0 |
| | | K₂WO₄ | [mg/kg-cat] | - | - | - | - | - | 240 | - | - |
| | | Na₂MoO₄ | [mg/kg-cat] | - | - | - | - | - | - | - | - |
| | | Mo₇O₂₄(NH₄)₆ | [mg/kg-cat] | - | - | - | 240 | 240 | - | 1000 | - |
| Catalyst Composition | Water | Volume | [mL] | 64 | 64 | 64 | 64 | 64 | 64 | 64 | 64 |
| | | W | [mg/kg-cat] | 46 | 5 | 466 | - | 60 | 1 | | - |
| | | Mo | [mg/kg-cat] | - | - | - | 0 | 0 | - | 0 | - |
| | | pH | [-] | 10.5 | 10.2 | 10.3 | 10.2 | 10.5 | 9.9 | 10.3 | 10.0 |
| | Sponge Cobalt Catalyst | Co | [mass%] | 55 | 55 | 55 | 55 | 55 | 55 | 55 | 55 |
| | | Al | [mass%] | 45 | 45 | 45 | 45 | 45 | 45 | 45 | 45 |
| | | W | [mg/kg-cat] | 104 | 88 | 534 | - | 90 | 149 | - | - |
| | | Mo | [mg/kg-cat] | - | - | - | 150 | 150 | - | 1000 | - |
| | | W/Co | [mol/mol] | 0.000061 | 0.000051 | 0.000311 | - | 0.000052 | 0.000087 | - | - |
| | | Mo/Co | [mol/mol] | - | - | - | 0.000167 | 0.000167 | - | 0.001117 | - |
| | | Mo/W | [mol/mol] | - | - | - | - | 3 | - | - | - |
| | | Grain Size | [mm] | 1-5 | 1-5 | 1-5 | 1-5 | 1-5 | 1-5 | 1-5 | 1-5 |
| Reaction Evaluation | Isophthalonitrile Ratio | | 1st Time | 0.0% | 0.6% | 31.3% | 1.0% | 0.6% | 3.8% | 2.6% | 0.6% |
| | | | 2nd Time | 0.0% | 0.4% | 2.5% | 0.7% | 0.5% | 2.7% | 1.9% | 1.3% |
| | | | 3rd Time | 0.3% | 0.7% | 14.6% | 1.4% | 0.8% | 1.9% | 1.9% | 1.1% |
| | | | 4th Time | 0.8% | 1.1% | 2.3% | 2.0% | 0.6% | 2.7% | 2.8% | 1.2% |
| | | | 5th Time | 0.7% | 3.2% | 1.7% | 3.7% | 1.3% | 5.5% | 4.0% | 6.3% |
| | Meta-xylenediamine Ratio | | 1st Time | 90.2% | 73.5% | 63.5% | 59.2% | 74.3% | 61.0% | 54.1% | 69.6% |
| | | | 2nd Time | 84.0% | 73.7% | 34.5% | 62.3% | 53.8% | 48.3% | 53.7% | 40.8% |
| | | | 3rd Time | 56.0% | 71.0% | 35.1% | 55.8% | 60.4% | 48.4% | 54.2% | 39.7% |
| | | | 4th Time | 42.6% | 58.2% | 41.0% | 55.6% | 68.8% | 42.9% | 51.6% | 39.9% |
| | | | 5th Time | 47.0% | 50.9% | 40.4% | 39.1% | 62.9% | 43.8% | 47.2% | 29.7% |

It is understood from the results in Fig. 2 that the ratio of isophthalonitrile in the reaction solution in Example 1 in which WO4 was adsorbed to the sponge cobalt catalyst was smaller than that in Comparative 1 in which no WO4 was adsorbed to the sponge cobalt catalyst. The isophthalonitrile left in the reaction solution was a remained isophthalonitrile without being reacted. The smaller amount of isophthalonitrile contained in the reaction solution means the more isophthalonitrile was reacted, that is, the catalyst activity was high. It is thus believed that the sponge cobalt catalyst of Example 1 had higher catalyst activity than the sponge cobalt catalyst of Comparative 1. Using each catalyst continuously made the difference more conspicuous. In the ratio of isophthalonitrile after five reactions, the ratio of isophthalonitrile was approximately 6% in Comparative 1, whereas the ratio of isophthalonitrile was approximately 1% in Example 1.

In addition to the results in Fig. 2, also in the ratio of meta-xylylenediamine obtained by hydrogenating isophthalonitrile, it is understood from the results in Fig. 3 that the amount of meta-xylylenediamine generated by using the sponge cobalt catalyst of Example 1 was larger than that generated by using the sponge cobalt catalyst of Comparative 1.

It is understood from the results of Figs. 4 and 5 that the catalyst activity after five reactions of Example 4 in which Mo₇O₂₄ was adsorbed to the sponge cobalt catalyst was higher than that of Comparative 1 in which no Mo₇O₂₄ was adsorbed to the sponge cobalt catalyst. Similar to the sponge cobalt catalyst of Example 1 in which WO₄ was adsorbed to the sponge cobalt catalyst, the sponge cobalt catalyst to which Mo₇O₂₄ was adsorbed exhibited high catalyst activity even in a long-term use. Further, the catalyst activity after five reactions of Example 5 in which WO₄ and Mo₇O₂₄ were adsorbed to the sponge cobalt catalyst was higher than those of Example 1 in which WO₄ was adsorbed to the sponge cobalt catalyst and Example 4 in which Mo₇O₂₄ was adsorbed to the sponge cobalt catalyst.

## Claims

1. A sponge cobalt catalyst composition comprising:
water;
an oxoacid; and
a sponge cobalt catalyst, wherein
the oxoacid comprises W or Mo, and
part or all of the oxoacid is adsorbed to the sponge cobalt catalyst.

2. The sponge cobalt catalyst composition according to claim 1, wherein a content of the oxoacid comprising W and adsorbed to the sponge cobalt catalyst is in a range from 5 mg to 1,200 mg, in terms of W, with respect to 1 kg of the sponge cobalt catalyst.

3. The sponge cobalt catalyst composition according to claim 2, wherein a content of the oxoacid comprising Mo and adsorbed to the sponge cobalt catalyst is in a range from 5 mg to 1,000 mg, in terms of Mo, with respect to 1 kg of the sponge cobalt catalyst.

4. The sponge cobalt catalyst composition according to claim 3, wherein the oxoacid is at least one selected from WO₄²⁻, MoO₄²⁻, Mo₇O₂₄⁶⁻, and Mo₈O₂₆⁴⁻.

5. The sponge cobalt catalyst composition according to claim 4, wherein a molar ratio (W/Co) of W comprised in the oxoacid adsorbed to the sponge cobalt catalyst to Co comprised in the sponge cobalt catalyst is in a range from 0.00001 to 0.0005.

6. The sponge cobalt catalyst composition according to claim 5, wherein a molar ratio (Mo/Co) of Mo comprised in the oxoacid adsorbed to the sponge cobalt catalyst to Co comprised in the sponge cobalt catalyst is in a range from 0.00001 to 0.01.

7. The sponge cobalt catalyst composition according to claim 6, wherein a molar ratio of Mo to W (Mo/W) comprised in the oxoacid adsorbed to the sponge cobalt catalyst is in a range from 1 to 10.

8. The sponge cobalt catalyst composition according to claim 7, wherein a content of cobalt comprised in the sponge cobalt catalyst is in a range from 30 mass% to 70 mass%.

9. The sponge cobalt catalyst composition according to claim 8, wherein a content of aluminum comprised in the sponge cobalt catalyst is in a range from 30 mass% to 70 mass%.

10. The sponge cobalt catalyst composition according to any one of claims 1 to 9, wherein the sponge cobalt catalyst composition is used in a reaction for hydrogenating nitrile.

11. A method for producing a sponge cobalt catalyst composition, the method comprising:
preparing an alloy comprising cobalt and aluminum;
removing the aluminum from the alloy to obtain a sponge cobalt catalyst;
immersing the sponge cobalt catalyst in water; and
adding an oxoacid salt comprising W or Mo to the water to adsorb the oxoacid to the sponge cobalt catalyst.
